# EUROPEAN PATENT APPLICATION

(11) **EP 4 008 265 A1**
(43) Date of publication of application: **08.06.2022**
(21) Application number: 21820075.6
(22) Date of filing: 14.09.2021
(51) Int. Cl.: A61B 8/00

(54) **ULTRASONIC DIAGNOSTIC DEVICE, ULTRASONIC PROBE, IMAGE GENERATION METHOD AND STORAGE MEDIUM**

(30) Priority: 10.10.2020 CN 202011079517
(71) Applicant: Cloudminds Robotics Co., Ltd., Shanghai 200245 (CN)
(72) Inventor: LUO, Lei, Shanghai 200245 (CN)
(74) Representative: Renaudo, Adrien Hanouar
(86) International application number: PCT/CN2021/118113
(87) International publication number: WO 2022/073410

(57) **Abstract**

Provided are an ultrasonic diagnostic device, an ultrasonic probe, a method for generating an image, and a storage medium. The ultrasonic diagnostic device (10) includes: an ultrasonic probe (101), and a main control assembly (102) connected with the ultrasonic probe (101); the ultrasonic probe (101) includes a probe main body (1011) and a pose detection assembly (1012); the pose detection assembly (1012) is configured to acquire pose data of the ultrasonic probe (101) in real time; the probe main body (1011) is configured to continuously scan a detection object to acquire corresponding ultrasonic images, and transmit the ultrasonic images and the corresponding pose data to the main control assembly (102); and the main control assembly (102) is configured to stitch, according to the pose data, the continuous ultrasonic images, so as to generate an ultrasonic stereo image of the detection object. By adopting the embodiments of the present disclosure, without professional knowledge, a general user may also acquire ultrasonic images that accurately reflect a lesion, and the usage difficulty of the ultrasonic diagnostic device is lowered.

## Description

### FIELD

The present disclosure relates to the technical field of ultrasonic waves, in particular to an ultrasonic diagnostic device, an ultrasonic probe, a method for generating an image, and a storage medium.

### BACKGROUND

For current ultrasonic diagnostic device, images acquired are 2D ultrasonic slice data. Professional doctors perform diagnosis on the basis of the 2D ultrasonic slice image. Saved data is also one or several 2D ultrasonic slice images.

However, a human body is a three-dimensional individual. Although a professional doctor may obtain, through techniques and experience, a 2D ultrasonic slice image that best reflects a disease, not all doctors can find a 2D ultrasonic slice image at the most ideal position in an ultrasonic inspection operation. For example, doctors working in town and village may rarely use or never use an ultrasound diagnostic device. If 2D ultrasonic slice images that accurately reflect lesions need to be acquired, a long time training of manipulation techniques with regard to each organ is required, and it is difficult for them to acquire correct 2D ultrasonic slice images only through remote guidance. Therefore, it is difficult to realize a remote diagnostic scenario of ultrasound, and ultrasound has not been able to be promoted on a large scale.

### SUMMARY

Some embodiments of the present disclosure aim to provide an ultrasonic diagnostic device, an ultrasonic probe, a method for generating an image, and a storage medium, so that without professional knowledge, a user may acquire an ultrasonic image that accurately reflects a lesion, and the difficulty in use of the ultrasonic diagnostic device is lowered.

Some embodiments of the present disclosure provide an ultrasonic diagnostic device, including: an ultrasonic probe, and a main control assembly connected with the ultrasonic probe; the ultrasonic probe includes a probe main body and a pose detection assembly arranged in the probe main body; the pose detection assembly is configured to acquire pose data of the ultrasonic probe in real time; the probe main body is configured to continuously scan a detection object to acquire corresponding ultrasonic images, and transmit the ultrasonic images and the corresponding pose data to the main control assembly; and the main control assembly is configured to stitch, according to the pose data, the continuous ultrasonic images, so as to generate an ultrasonic stereo image of the detection object.

Some embodiments of the present disclosure further provide an ultrasonic probe including: a probe main body and a pose detection assembly; the pose detection assembly is configured to acquire pose data of the ultrasonic probe in real time; the probe main body is configured to continuously scan a detection object to acquire ultrasonic images corresponding to the pose data, and transmit the ultrasonic images and the corresponding pose data to the main control assembly; and the main control assembly is configured to stitch, according to the pose data, the continuous ultrasonic images, so as to generate an ultrasonic stereo image of the detection object.

Some embodiments of the present disclosure further provide a method for generating an image, which is applied to an ultrasonic diagnostic device and includes: when an ultrasonic probe being operated is detected, acquiring pose data containing the ultrasonic probe and ultrasonic images corresponding to the pose data; and stitching, according to the pose data, the continuous ultrasonic images, so as to generate an ultrasonic stereo image of a detection object.

Some embodiments of the present disclosure further provide a computer-readable storage medium which stores a computer program. The computer program, when executed by a processor, implements the method for generating an ultrasonic image. In the embodiments of the present disclosure, the ultrasonic probe includes the probe main body and the pose detection assembly arranged in the probe main body; the pose detection assembly is able to acquire a pose of the probe main body in real time; the probe main body is configured to continuously scan the detection object; and the main control assembly is able to acquire the pose data of the probe main body and the corresponding ultrasonic images in real time, and thus is able to stitch, according to the pose data, the continuously acquired ultrasonic images to form the ultrasonic stereo image. Since the ultrasonic stereo image is generated, which is a three-dimensional image that can display more details of the detection object, the accuracy of the detection object generated by the ultrasonic diagnostic device is improved. The user may read the ultrasonic stereo image as needed without professional ultrasonic operation knowledge, thus adding the usage scenario of the ultrasonic diagnostic device.

### BRIEF DESCRIPTION OF THE DRAWINGS

One or more embodiments are exemplified by the corresponding accompanying drawings. These exemplified descriptions do not constitute a limitation to the embodiments. Elements with the same reference numerals in the accompanying drawings are shown as similar elements. The drawings in the accompanying drawings do not constitute scaling restrictions unless otherwise stated.
FIG. 1 is a structural block diagram of an ultrasonic diagnostic device according to a first embodiment of the present disclosure;
FIG. 2 is a schematic diagram of an ultrasonic probe in an ultrasonic diagnostic device according to a second embodiment of the present disclosure;
FIG. 3 is a structural block diagram of a main control assembly according to a first embodiment of the present disclosure;
FIG. 4 is a schematic diagram of a moving direction of an ultrasonic probe according to a second embodiment of the present disclosure;
FIG. 5 is a schematic diagram of arrangement of ultrasonic images according to a second embodiment of the present disclosure;
FIG. 6 is another schematic diagram of arrangement of ultrasonic images according to a second embodiment of the present disclosure;
FIG. 7 is a schematic diagram of an ultrasonic stereo image according to a second embodiment of the present disclosure;
FIG. 8 is a structural block diagram of an ultrasonic diagnostic device according to a second embodiment of the present disclosure;
FIG. 9 is a schematic diagram of an ultrasonic image of a specified section according to a second embodiment of the present disclosure;
FIG. 10 is a structural block diagram of a main control assembly according to a third embodiment of the present disclosure;
FIG. 11 is a schematic diagram of arrangement of ultrasonic images according to a third embodiment of the present disclosure;
FIG. 12 is a structural block diagram of an ultrasonic probe according to a fourth embodiment of the present disclosure; and
FIG. 13 is a flowchart of a method for generating an ultrasonic image according to a fifth embodiment of the present disclosure.

### DETAILED DESCRIPTION OF EXAMPLE EMBODIMENTS

In order to make the objectives, technical solutions and advantages of the present disclosure clearer, some embodiments of the present disclosure is further described below in detail with reference to accompanying drawings and embodiments. Those of ordinary skill in the art can understand that in the various embodiments, many technical details are presented in order to make the present disclosure better understood by readers. However, the technical solutions claimed in the present disclosure can also be implemented without these technical details and various changes and modifications based on the embodiments. All the following embodiments are divided for the convenience of description and shall not constitute any restriction on the specific implementation modes of the present disclosure. All the embodiments can be combined and referenced with each other with no conflicts.

A first embodiment of the present disclosure relates to an ultrasonic diagnostic device. The ultrasonic diagnostic device 10 includes: an ultrasonic probe 101, and a main control assembly 102 connected with the ultrasonic probe 101; the ultrasonic probe 101 includes a probe main body 1011 and a pose detection assembly 1012 arranged in the probe main body 1011; the pose detection assembly 1012 is configured to acquire pose data of the probe main body 1011 in real time; the probe main body 1011 is configured to continuously scan a detection object to acquire corresponding ultrasonic images, and transmit the ultrasonic images and the corresponding pose data to the main control assembly 1012; and the main control assembly 1012 is configured to stitch, according to the pose data, the continuous ultrasonic images, so as to generate an ultrasonic stereo image of the detection object.

In the embodiments of the present disclosure, the ultrasonic probe includes the probe main body and the pose detection assembly arranged in the probe main body; the pose detection assembly is able to acquire a pose of the probe main body in real time; the probe main body is configured to continuously scan the detection object; and the main control assembly is able to acquire the pose data of the probe main body and the corresponding ultrasonic images in real time, and thus is able to stitch, according to the pose data, the continuously acquired ultrasonic images to form the ultrasonic stereo image. Since the ultrasonic stereo image is generated, which is a three-dimensional image that can display more details of the detection object, the accuracy of the detection object generated by the ultrasonic diagnostic device is improved. The user can read the ultrasonic stereo image as needed without professional ultrasonic operation knowledge, thus adding the usage scenario of the ultrasonic diagnostic device.

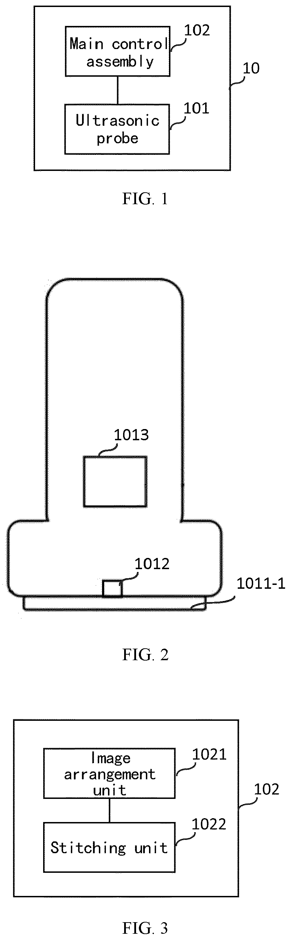

A second embodiment of the present disclosure relates to an ultrasonic diagnostic device. The present embodiment is a specific introduction of the ultrasonic diagnostic device in the first embodiment. The ultrasonic diagnostic device is introduced below in combination with FIG. 1 and FIG. 2.

As shown in FIG. 1, the ultrasonic diagnostic device 10 includes a probe main body 1011, and a main control assembly 102 connected with the probe main body 1011. The structure of the probe main body 1011 is as shown in FIG. 2 and includes a probe main body 1011 and a pose detection assembly 1012 arranged in the probe main body 1011.

Specifically, the pose detection assembly 1012 may be a motion sensor. The motion sensor may be arranged at a position close to a scanning surface of the probe main body 1011. A processor 1013 as shown in FIG. 2 may be also arranged in the probe main body 101 and is configured to acquire pose data collected by the motion sensor and transmit the pose data to the main control assembly 102. The probe main body 1011 may include: a two-dimensional probe or a three-dimensional probe. The probe of the probe main body 1011 is shown by 1011-1 in FIG. 2. The main control assembly 102 is not shown in FIG. 2. The main control assembly 102 may include a processor, such as a computer device.

An operating process of the ultrasonic diagnostic device is as follows: when the probe main body 1011 is initiated, the pose detection assembly 1012 located in the probe main body 1011 is also synchronously initiated; the pose detection assembly 1012 starts to detect the pose data of the probe main body 1011; the pose data may be transmitted to the probe main body 1011, and the probe main body 1011 transmits the pose data to the main control assembly 102; and the probe main body 1011 continuously scans a detection object to obtain continuous ultrasonic images and transmit the continuous ultrasonic images to the main control assembly 102 in real time. By means of setting a synchronous clock, the probe main body 1011 and the pose detection assembly 1012 are synchronously initiated.

A user may place the ultrasonic probe 101 on a part to be detected of the detection object. After the probe main body 1011 contacts the detection object, prompt information of initiation of the probe main body 1011 may be sent to the processor in the probe main body 1011. When the probe main body 1011 acquires the prompt information, the pose detection assembly 1012 may be initiated to acquire the pose data of the probe main body 1011 in real time. A collection frequency of the pose detection assembly 1012 may be set. If the frequency is higher, a subsequently generated ultrasonic stereo image is more accurate.

It should be noted that a time tag that represents scanning time may be added, according to the chronological order of the scanning time of the probe main body 1011, for each ultrasonic image. Similarly, a time tag that represents pose acquisition time may be added for each of the pose data. The pose data of the probe main body 1011 corresponding to the ultrasonic image acquired at the same time may be searched through the time tag.

In one example, the main control assembly 102 includes an image arrangement unit 1021 and a stitching unit 1022; the image arrangement unit 1021 is configured to perform the following arrangement processing on each of the pose data and corresponding ultrasonic data; acquire a probe position and a probe angle of the probe main body in a preset three-dimensional spatial coordinate system according to the pose data; and place, according to the probe angle, the ultrasonic image corresponding to the probe angle on the probe position; and the stitching unit is configured to stitch the ultrasonic images after being arranged to form the ultrasonic stereo image. The structural block diagram of the main control assembly 102 is shown in FIG. 3.

The image arrangement unit 1021 further includes a position acquisition sub-unit and an angle acquisition sub-unit; the position acquisition sub-unit is configured to process each of the pose data as follows: when a change of the position of the probe main body is detected, acquiring a relative position datum of the current probe main body relative to the probe main body of a previous frame according to the pose datum of the current fame and the pose datum of the previous frame, and determining a probe position corresponding to the current frame according to the relative position datum and the probe position corresponding to the previous frame. The angle acquisition sub-unit is configured to process each of the pose data as follows: when a change of the angle of the ultrasonic probe is detected, acquiring a relative angle datum of the current probe main body relative to the probe main body of the previous frame according to the pose datum of the current fame and the pose datum of the previous frame, and determining a probe angle corresponding to the current frame according to the relative angle datum and the probe angle corresponding to the previous frame.

Specifically, the pose detection assembly 1012 may include a motion sensor, a gravity sensor, and a geomagnetic sensor. The pose data of the probe main body 1011 collected by the pose detection assembly include: a position coordinate and an angle coordinate of movement relative to a probe main body reference system. The three-dimensional spatial coordinate system may be preset. Any position in the three-dimensional spatial coordinate system may be taken as a corresponding initial probe position of the probe main body 1011 in the three-dimensional spatial coordinate system, and any angle may also be taken as an initial probe angle in the preset three-dimensional spatial coordinate system. The pose data under the probe main body reference system may be converted into data under the preset three-dimensional spatial coordinate system by means of an initial position of the probe main body 1011 relative to the probe main body reference system and the set initial probe position, as well as an initial angle of the probe main body 1011 relative to the probe main body reference system and the set initial probe angle.

A relative position change of the probe main body 1011 may be acquired by means of a difference between the pose data of the current frame and the pose data of the moment before the current frame, and the probe position of the probe main body of the current frame may be determined according to the relative position change and the coordinates of the probe main body of the previous frame in the three-dimensional spatial coordinate system. Similarly, an angle change of the probe main body 1011 may also be acquired, and the probe angle of the probe main body of the current frame is determined according to the relative angle change and the probe angle of the probe main body of the previous frame.

For example, the initial probe position of the probe main body is set as (x0, y0, z0) in the three-dimensional spatial coordinate system, and the probe angle of the probe main body of an initial frame t0 is set as (α0, β0, γ0); the pose data under the initial frame t0 include: an initial position coordinate (x0', y0', z0') and an initial angle (α0', β0', γ0'), i.e., (x0', y0', z0') corresponds to (x0, y0, z0) under the three-dimensional spatial coordinate system, and (α0', β0', γ0') corresponds to (α0, β0, γ0) under the three-dimensional spatial coordinate system. A position coordinate (xl', yl', zl') of the probe main body of a frame t1 under a probe main body coordinate system is acquired, and the relative position datum of the probe main body of the frame t1 relative to the probe main body of the previous frame is (△x, △y, △z), where △x=x1'- x0', △y =y1'- y0', △z= z1'- z0', so the probe position of the probe main body of the frame t1 is (x0+△x, y0+△y, z0+△z); and similarly, a relative change in the probe angle between the frame t1 and the frame t0 may be acquired, and the corresponding probe angle in the frame t1 is determined.

The probe main body continuously scans the detection object; each ultrasonic image is placed, according to the probe angle of the ultrasonic probe, at the probe position corresponding to the three-dimensional spatial coordinate system; and after it is detected that the ultrasonic probe completes the scanning, each ultrasonic image in the three-dimensional spatial coordinate system may be stitched to generate the ultrasonic stereo image.

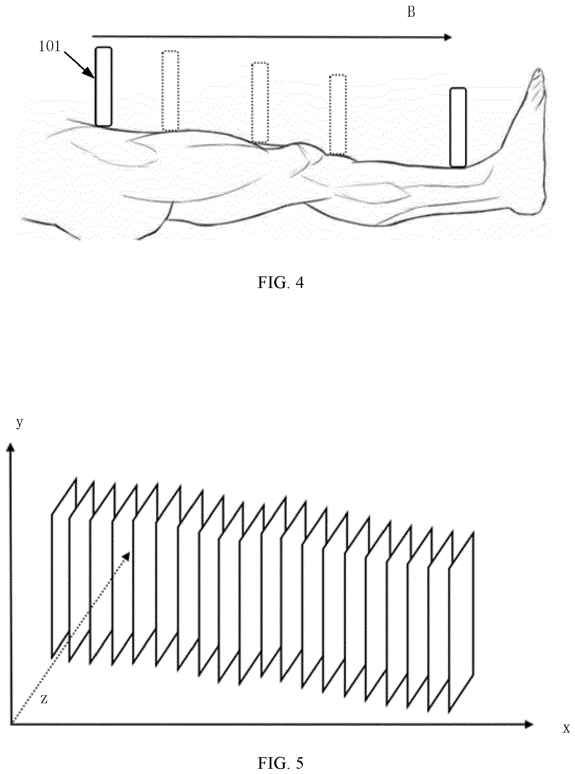

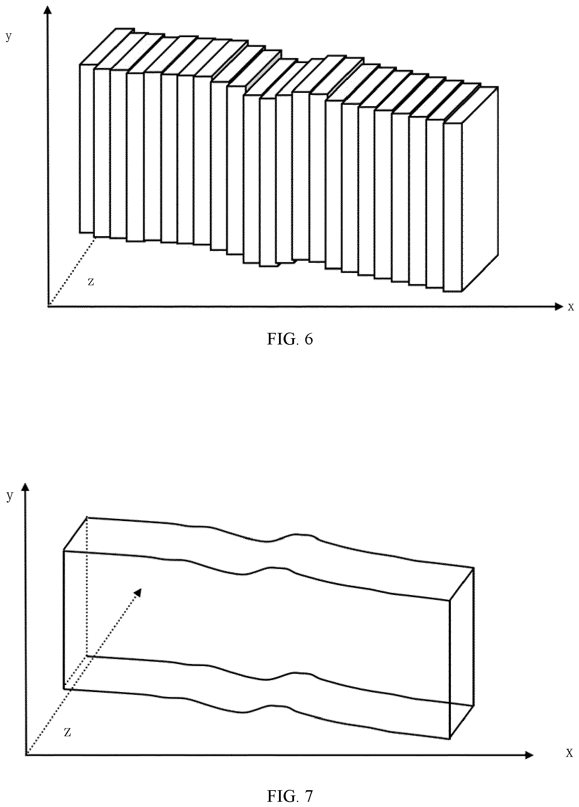

For example, the probe main body may scan a human leg according to a moving direction B as shown in FIG. 4. The human leg is the detection object. If the probe main body is a 2D probe, each ultrasonic image is arranged as follows: placing the ultrasonic image at the corresponding probe position according to the probe angle. After it is detected that the probe main body completes the scanning, an ultrasonic image arrangement diagram as shown in FIG. 5 is formed. Each ultrasonic image is an optical coherence tomography of the leg. A section interval will also be different according to a change in a moving speed of the probe main body 1011. The probe main body usually has a high sampling frequency, so an ultrasonic image arrangement diagram with a very small actual interval may be formed. The ultrasonic images arranged as shown in FIG. 5 are stitched to form the ultrasonic stereo image as shown in FIG. 7. If the moving speed of the ultrasonic probe is lower, a three-dimensional point cloud obtained is denser, and the formed ultrasonic stereo image is more accurate.

If the ultrasonic probe is a 3D probe, each frame of ultrasonic image has a certain width, and each scanned frame of ultrasonic image is a cubic slice with a certain width and is one three-dimensional optical coherence tomography, which is in a form as shown in Fig 6. Since width information is acquired at each time, if the probe main body 1011 moves slowly, there will be an image overlap part in two adjacent sampling operations, and the data of the overlap part shall be identical. Therefore, after each ultrasonic image is stitched, one stereo semipermeable dense point cloud graph is obtained, thus forming three-dimensional point cloud data and obtaining an ultrasonic stereo image of the leg, as shown in FIG. 7.

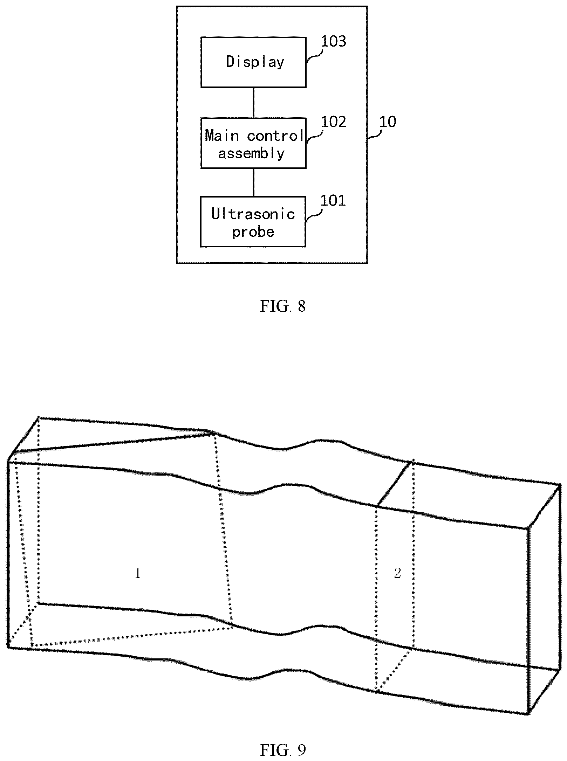

In one example, the ultrasonic diagnostic device 10 further includes: a display 103 connected with the main control assembly 102. The display 103 is configured to display the ultrasonic stereo image. The main control assembly 102 is further configured to slice, when an instruction that instructs a specified section to be display is detected, the ultrasonic stereo image, generate an ultrasonic image of the specified section, and transmit the ultrasonic image to the display 103. The structure of the ultrasonic diagnostic device may be further as shown in FIG. 8.

Specifically, the display 103 may be an ordinary display, or may be a holographic image display. The display displays the ultrasonic stereo image. If the user needs to observe the ultrasonic image of the specified section, the user may input information of the specified section through an input device. The main control assembly 102 slices the ultrasonic stereo image after receiving the instruction, generates the ultrasonic image of the specified section, and transmits the ultrasonic image to the display 103. The display displays the ultrasonic image of the specified section. The specified section is a section 1 and a section 2 as shown in FIG. 9.

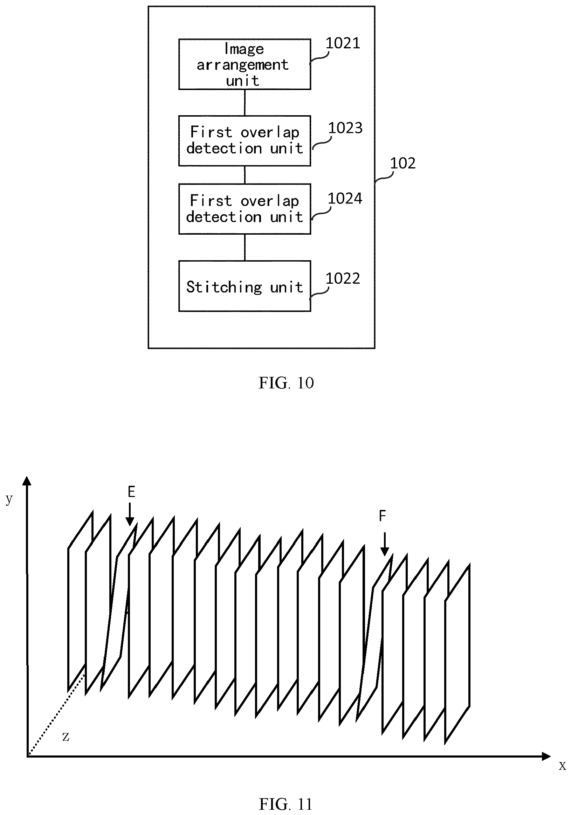

A third embodiment of the present disclosure relates to an ultrasonic diagnostic device. The present embodiment is an improvement of the first embodiment or the second embodiment. A main improvement is that the main control assembly 102 of the present embodiment further includes a first overlap detection unit 1023. The structural block diagram of the main control assembly 102 is as shown in FIG. 10.

The first overlap detection unit 1023 is configured to process the ultrasonic images after being arranged as follows: detecting whether an overlap image is present between the current ultrasonic image and the ultrasonic image of the previous frame; and if yes, re-acquiring an ultrasonic image of the current frame, or updating the ultrasonic image of the current frame according to the overlap image.

Since the movement of the probe main body 1011 is unsteady, there will be a phenomenon that an ultrasonic image tilts relative to other ultrasonic images in the ultrasonic image arrangement diagram obtained according to the probe angle, i.e., the ultrasonic image E and the ultrasonic image F as shown in FIG. 11. In the present embodiment, the first overlap detection unit detects a tilting phenomenon of an ultrasonic image. If there is a tilted image as shown in FIG. 11, the ultrasonic image E inevitably overlaps the ultrasonic images of the previous and latter frames. Based on this principle, each ultrasonic image after being is detected: detecting whether an overlap image is presented between the current ultrasonic image and the ultrasonic image of the previous frame, and if yes, re-acquiring an ultrasonic image of the current frame, or updating the ultrasonic image of the current frame according to the overlap image.

If there is an overlap image, it is indicated that the ultrasonic image of the current frame is a tilted image. If the scanning frequency of the probe main body is greater than a preset frequency, the tilted ultrasonic image may not be processed. If the scanning frequency is less than the preset frequency, it may be indicated that the scanning frequency is increased to re-scan a specified scanning region, an ultrasonic image of the current frame is re-acquired. The specified scanning region may also be an organ to be detected. The image of the specified scanning region may be an organ image corresponding to the detection object, and the organ image may be projected at the position of the organ to be detected of the detection object. New information after operation may also be generated according to information of the overlap image by using an algorithm and is used as an ultrasonic image of this frame.

It can be understood that the ultrasonic image of the initial frame may be a normal image by default.

The main control assembly 102 further includes a second overlap detection unit 1024. The second overlap detection unit 1024 is configured to: after it is detected that the probe main body completes the scanning, if an overlap part between a coverage region formed by all the acquired ultrasonic images and the preset specified scanning region is smaller than the specified scanning region, output first error indication information; and/or, if it is detected that the ultrasonic images include a fuzzy image, output second error indication information.

Specifically, if it is detected that the probe main body 1011 leaves a surface of the detection object, it is determined that the ultrasonic probe completes the scanning. Before the stitching of each ultrasonic image, it may be detected whether the overlap part between the coverage region formed by all the ultrasonic images and the specified scanning region is smaller than the specified scanning region; if yes, it is indicated that there is a region not scanned, and the first error indication information may be output. In addition, it may also be detected whether the acquired ultrasonic images include the fuzzy image; if yes, the second error indication information is output. The first error indication information is configured to indicate that there is a missing region. The second error indication information is configured to indicate that there is a fuzzy image. The output may be realized by a voice. The user may re-acquire an accurate ultrasonic image by means of outputting the first error indication information or the second error indication information, and the accuracy of detection is improved.

The division of the steps of the various methods above is only for the clarity of description. When implemented, they can be combined into one step or some steps can be split into multiple steps, as long as they include the same logical relationship and all fall within the scope of protection of this patent. Adding insignificant modifications to algorithms or processes or introducing insignificant designs, but not changing the core designs of the algorithms and processes, falls within the scope of protection of this patent.

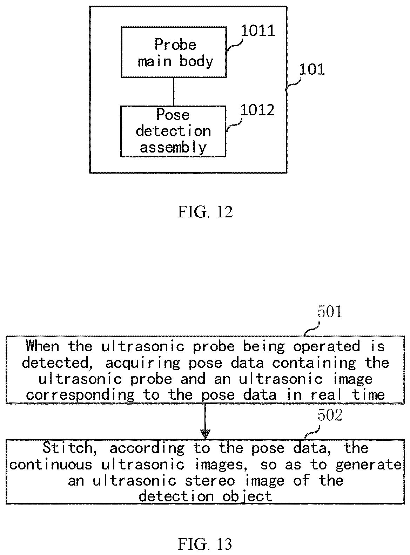

The fourth embodiment of the present disclosure relates to an ultrasonic probe. The structural block diagram of the ultrasonic probe 101 is as shown in FIG. 12, including a probe main body 1011 and a pose detection assembly 1012; the pose detection assembly 1012 is configured to acquire pose data of the ultrasonic probe 1011 in real time; the probe main body is configured to continuously scan a detection object to acquire ultrasonic images corresponding to the pose data, and transmit the ultrasonic images and the corresponding pose data to the main control assembly; and the main control assembly 1012 is configured to stitch, according to the pose data, the continuous ultrasonic images, so as to generate an ultrasonic stereo image of the detection object.

The fifth embodiment of the present disclosure relates to a method for generating an ultrasonic image, which is applied to an ultrasonic diagnostic device. The process is as shown in FIG. 13.

At step 501: when an ultrasonic probe being operated is detected, pose data containing the ultrasonic probe and an ultrasonic image corresponding to the pose data are acquired in real time.

Specifically, when a probe main body is initiated, a pose detection assembly located in the probe main body is also synchronously initiated; the pose detection assembly starts to detect the pose data of the probe main body; the pose data may be transmitted to the probe main body, and the probe main body transmits the pose data to the main control assembly; and the probe main body continuously scans a detection object to obtain continuous ultrasonic images and transmit the images to the main control assembly in real time. By means of setting a synchronous clock, the probe main body and the pose detection assembly are synchronously initiated.

A user may place the ultrasonic probe on a part to be detected of the detection object. After the probe main body contacts the detection object, the probe main body can send prompt information of initiation of the probe main body to the processor in the probe main body. When the processor in the probe main body acquires the prompt information, the pose detection assembly can be initiated to in real time acquire the pose data of the probe main body. A collection frequency of the pose detection assembly may be set. If the frequency is higher, a subsequently generated ultrasonic stereo image is more accurate. The pose data of the probe main body acquired by the pose detection assembly include: a position coordinate of movement relative to a probe main body reference system and an angle coordinate relative to the probe main body reference system.

At step 502: the continuous ultrasonic images are stitched according to the pose data, so as to generate an ultrasonic stereo image of the detection object.

Specifically, a probe position and a probe angle of the probe main body in a preset three-dimensional spatial coordinate system are acquired according to the pose data; the ultrasonic image corresponding to the probe angle is placed on the probe position according to the probe angle; and a stitching unit is configured to stitch the ultrasonic images after being arranged, so as to form the ultrasonic stereo image. The process of acquiring the probe position specifically includes: when a change of the position of the probe main body is detected, acquiring a relative angle datum of the current probe main body relative to the probe main body of the previous frame according to the pose datum of the current fame and the pose datum of the previous frame, and determining a probe angle corresponding to the current frame according to the relative angle datum and the probe position corresponding to the previous frame. The process of acquiring the probe angle includes: when a change of the angle of the ultrasonic probe is detected, acquiring a relative angle datum of the current probe main body relative to the previous frame of probe main body according to the pose datum of the current fame and the pose datum of the previous frame, and determining a probe angle corresponding to the current frame according to the relative angle datum and the probe angle corresponding to the previous frame.

The probe main body continuously scans the detection object; each ultrasonic image is placed, according to the probe position of the ultrasonic probe, in the three-dimensional spatial coordinate system; and after it is detected that the ultrasonic probe completes the scanning, each ultrasonic image in the three-dimensional spatial coordinate system may be stitched to generate the ultrasonic stereo image. The main control assembly in the ultrasonic diagnostic device may include a memory and a processor. The memory is in communication connection with the at least one processor. The memory stores an instruction that may be executed by the at least one processor. The instruction is executed by the at least one processor to cause the at least one processor to be able to implement the above method for generating an ultrasonic image.

The memory and the processor are connected by a bus. The bus may include any number of interconnected buses and bridges. The bus links one or more processors with various circuits of the memory together. The bus may also link various other circuits such as peripherals, voltage regulators and power management circuits, which are well known in the art and, therefore, will not be further described herein. A bus interface provides an interface between the bus and a transceiver. The transceiver may be one element or a plurality of elements, such as a plurality of receivers and transmitters to provide units for communicating with various other devices on a transmission medium. Data processed by the processor is transmitted on a wireless medium through an antenna, and further, the antenna also receives the data and transmits the data to the processor.

The processor is responsible for managing the bus and performing general processing, and can also provide various functions including timing, peripheral interfaces, voltage regulation and power management, and other control functions. The memory can be used to store data used by the processor when performing operations.

The sixth embodiment of the present disclosure relates to a computer-readable storage medium which stores a computer program. The computer program, when executed by a processor, implements the method for generating an ultrasonic image.

Those skilled in the art can understand that all or part of the steps in the method of the above embodiment can be completed by a program that instructs related hardware. This program is stored in a storage medium and includes a number of instructions configured to enable one device (which may be a single-chip microcomputer, a chip, and the like) or a processor to execute all or part of the steps of the methods described in the various embodiments of the present disclosure. The aforementioned storage media include: a USB flash disk, a mobile hard disk, a read-only memory (ROM), a random access memory (RAM), a magnetic disk or an optical disk, and other media that can store program codes.

Those of ordinary skill in the art can understand that the above-mentioned implementation modes are specific embodiments for realizing the present disclosure, and in actual applications, various changes can be made in form and details without departing from the spirit and scope of the present disclosure.

## Claims

1. An ultrasonic diagnostic device, comprising: an ultrasonic probe, and a main control assembly connected with the ultrasonic probe, wherein,
the ultrasonic probe comprises a probe main body and a pose detection assembly; the pose detection assembly is configured to acquire pose data of the probe main body in real time; the probe main body is configured to continuously scan a detection object to acquire corresponding ultrasonic images, and transmit the ultrasonic images and the corresponding pose data to the main control assembly; and
the main control assembly is configured to stitch, according to the pose data, the continuous ultrasonic images, so as to generate an ultrasonic stereo image of the detection object.

2. The ultrasonic diagnostic device according to claim 1, wherein the main control assembly comprises an image arrangement unit and a stitching unit;
the image arrangement unit is configured to perform the following arrangement processing on each of the pose data and corresponding ultrasonic data: acquiring a probe position and a probe angle of the probe main body in a preset three-dimensional spatial coordinate system according to the pose data, and placing, according to the probe angle, the ultrasonic image corresponding to the probe angle on the probe position; and
the stitching unit is configured to stitch the ultrasonic images after being arranged to form the ultrasonic stereo image.

3. The ultrasonic diagnostic device according to claim 2, wherein the image arrangement unit further comprises a position acquisition sub-unit and an angle acquisition sub-unit;
the angle acquisition sub-unit is configured to process each of the pose data as follows: when a change of the position of the probe main body is detected, acquiring a relative position datum of a current probe main body relative to the probe main body of a previous frame according to pose datum of a current fame and pose datum of the previous frame, and determining a probe position corresponding to the current frame according to the relative angle datum and the probe position corresponding to the previous frame;
the angle acquisition sub-unit is configured to process each of the pose data as follows: when a change of the angle of the ultrasonic probe is detected, acquiring a relative angle datum of the current probe main body relative to the probe main body of the previous frame according to the pose datum of the current fame and the pose datum of the previous frame, and determining a probe angle corresponding to the current frame according to the relative angle datum and the probe angle corresponding to the previous frame.

4. The ultrasonic diagnostic device according to claim 2 or 3, wherein the main control assembly further comprises a first overlap detection unit;
the first overlap detection unit is configured to process the ultrasonic images after being arranged as follows: detecting whether an overlap image between the current ultrasonic image and the ultrasonic image of the previous frame is exited; and if yes, re-acquiring an ultrasonic image of the current frame, or updating the ultrasonic image of the current frame according to the overlap image.

5. The ultrasonic diagnostic device according to any one of claims 1 to 4, wherein the main control assembly further comprises a second overlap detection unit;
the second overlap detection unit is configured to: after the probe main body completing a scanning is detected, if an overlap part between a coverage region formed by all the acquired ultrasonic images and a preset specified scanning region is smaller than a specified scanning region, output first error indication information; and/or,
if the ultrasonic images including a fuzzy image is detected, output second error indication information.

6. The ultrasonic diagnostic device according to any one of claims 1 to 5, wherein the ultrasonic diagnostic device further comprises a display connected with the main control assembly;
the display is configured to display the ultrasonic stereo image;
the main control assembly is further configured to slice, when an instruction instructing a specified section to be display is detected, the ultrasonic stereo image, generate an ultrasonic image of the specified section, and transmit the ultrasonic image of the specified section to the display.

7. The ultrasonic diagnostic device according to any one of claims 1 to 6, wherein the probe main body comprises a two-dimensional probe or a three-dimensional probe;
the pose detection assembly comprises a motion sensor.

8. The ultrasonic diagnostic device according to any one of claims 1 to 7, wherein the pose detection assembly is located in the probe main body.

9. An ultrasonic probe, comprising: a probe main body and a pose detection assembly; wherein,
the pose detection assembly is configured to acquire pose data of the probe main body in real time;
the probe main body is configured to continuously scan a detection object to acquire ultrasonic images corresponding to the pose data, and transmit the ultrasonic images and the corresponding pose data to the main control assembly; and the main control assembly is configured to stitch, according to the pose data, the continuous ultrasonic images, so as to generate an ultrasonic stereo image of the detection object.

10. A method for generating an image, applied to the ultrasonic diagnostic device according to any one of claims 1 to 8 and comprising:
when an ultrasonic probe being operated is detected, acquiring pose data containing the ultrasonic probe and an ultrasonic image corresponding to the pose data in real time; and
stitching, according to the pose data, the continuous ultrasonic images, so as to generate an ultrasonic stereo image of the detection object.

11. A computer-readable storage medium, storing a computer program, wherein the computer program, when executed by a processor, implements the method for generating an ultrasonic image according to claim 10.
